# EUROPÄISCHE PATENTSCHRIFT

(11) **EP 0 542 004 B1**
(45) Veröffentlichungstag und Bekanntmachung des Hinweises auf die Patenterteilung: **09.08.1995**
(21) Anmeldenummer: 92118045.1
(22) Anmeldetag: 22.10.1992
(51) Int. Cl.: A61B 17/58

(54) **Stützmittel für die menschliche Wirbelsäule**
Support plate for the human spine
Plaque support de rachis

(30) Priorität: 13.11.1991 DE 9114118 U
(43) Veröffentlichungstag der Anmeldung: 19.05.1993
(73) Patentinhaber: Howmedica GmbH, D-24232 Schönkirchen (DE)
(72) Erfinder: Procter, Philip, Weybridge, Surrey KT15 3DJ (GB); Bird, Nicola, Nr. Uxbridge, South Bucks. UB9 5JE (GB); Harder, Hans Erich, W-2316 Probsteierhagen (DE)
(74) Vertreter: Patentanwälte Hauck, Graalfs, Wehnert, Döring, Siemons

(56) Entgegenhaltungen:
- EP-A- 0 290 138
- EP-A- 0 410 309
- WO-A-90/04366
- WO-A-90/13266
- WO-A-92/11819
- CH-A- 611 147
- FR-A- 2 405 706
- GB-A- 2 125 295
- US-A- 2 980 110

## Beschreibung

Die Erfindung bezieht sich auf ein Stützmittel für die Korrektur und/oder die Stabilisierung traumatisierter oder defizienter Wirbel der menschlichen Wirbelsäule nach dem Oberbegriff des Anspruchs 1.

Zur Reposition von gesunden und/oder frakturierten Wirbeln ist aus dem DE-GM 87 11 317 bekannt, sogenannte Schanz'sche Schrauben über die Pedikel in den Wirbelkörper einzuschrauben. Die Schanz'schen Schrauben werden mit einer Stützvorrichtung verbunden, welche eine Verstellung der Schanz'schen Schrauben bezüglich aller drei Freiheitsgrade ermöglichen. Die bekannte Stützvorrichtung ist indessen nicht in der Lage, mehr als einen Wirbel zu überbrücken oder etwa im Fall von sogenannten Etagenbrüchen eine multisegmentäre Versorgung zu gewährleisten. Für Impalantationszwecke müssen die Schanz'schen Schrauben nach einer Festlegung in der Stützvorrichtung gekürzt werden. Sie weisen daher an den Enden Grate auf. Wegen des nicht unerheblichen Platzbedarfs kann die bekannte Stützvorrichtung nicht im oberen Bereich der Wirbelsäule, insbesondere im Halswirbelbereich, eingesetzt werden.

Mit Hilfe eines sogenannten Harrington-Stabes, an dem Haken oder Pedikelschrauben anbringbar sind, lassen sich mehrere Wirbel überbrücken. Ein Harrington-Stab kann jedoch nicht an die jeweilige Form der Wibelsäule im applizierten Bereich individuell angepaßt werden. Es ist daher auch schon bekanntgeworden, einen Gewindedraht zu verwenden, der mit Pedikelschrauben zusammenwirkt.Diese bekannte Stützvorrichtung ermöglicht jedoch wegen ungenügender Festigkeit keine primärstabile Abstützung. Sie kann daher im wesentlichen nur verwendet werden für in sich stabile Wirbelsäulen, deren Verlauf aus orthopädischen Gründen korrigiert werden soll.

Aus dem DE-GM 88 02 112 ist eine Stützvorrichtung für die menschliche Wirbelsäule bekanntgeworden, bei der zwischen benachbarten Pedikelschrauben ein sogenanntes Spannschloß angeordnet ist, dessen Enden Klemmflächen aufweisen zwecks Anklemmung an den Kopf einer Pedikelschraube. Durch Veränderung des axialen Abstandes der Bolzen zueinander, zum Beispiel mit Hilfe einer Gewindehülse, lassen sich daher die Anbringungspunkte der Pedikelschrauben verstellen. Gleichzeitig ermöglichen die Bolzen eine Rotation der Pedikelschrauben um die Achse der Bolzen um einen gewünschten Winkel. Die Pedikelschrauben können darüber hinaus um eine Achse senkrecht zu ihrer Achse gegenüber den Verbindungspunkten verschwenkt und in der eingenommenen Position festgelegt werden. Die Pedikelschraube kann daher im Raum jede gewünschte Lage einnehmen und in dieser Lage festgesetzt werden. Die bekannte Stützvorrichtung ermöglicht daher das Einschrauben von Pedikelschrauben in jeden gewünschten Wirbel, um damit gewünschte Wirbel eines Wirbelsäulenabschnitts gegeneinander festzulegen. Eine derartige Stützvorrichtung ist daher für eine primäre Stabilisierung der Wirbelsäule, etwa im Fall von Frakturen eines oder mehrerer Wirbel geeignet. Aber auch diese Stützvorrichtung, die dorsal implantiert wird, was für den Patienten eine relativ geringe Belastung darstellt, ist für den oberen Wirbelsäulenbereich, insbesondere für die Halswirbel nicht geeignet.

Aus der CH-A-611 147 ist eine osteosynthetische Kompressionsplatte bekannt, bei der in Längsrichtung der Platte Löcher für Senkknochenschrauben vorgesehen sind. Zwischen den Schraublöchern sind kleinere kreisrunde Löcher in Querreihen angeordnet dergestalt, daß über die ganze Plattenlänge eine annähernd gleiche Biegesteifigkeit erzielt wird.

Aus der EP-A-0 410 390 ist eine Stabilisierungsplatte für Knochenwirbel gemäß dem Oberbegriff des unabhängigen Anspruchs 1 bekannt geworden, die als Leiste geformt ist mit einer Reihe von Langlöchern. Zwischen den Langlöchern sind Einschnitte vorgesehen, um die Leiste dem gewünschten Verlauf des Knochens, z.B. der Wirbelsäule anzupassen.

Der Erfindung liegt die Aufgabe zugrunde, ein Stützmittel für die Korrektur und/oder die Stabilisierung traumatisierter oder defizienter Wirbel der menschlichen Wirbelsäule zu schaffen, welches im oberen Wirbelsäulenbereich eingesetzt werden kann.

Diese Aufgabe wird gelöst durch die Merkmale des Anspruchs 1.

Die Erfindung geht von einer anterior an mindestens zwei benachbarten Wirbeln bzw. Wirbelkörpern anbringbaren länglichen Platte aus, die aus verformbarem, körperverträglichem Material besteht. Sie weist Schraubenlöcher auf zur Aufnahme von in die Wirbel bzw. Wirbelkörper einschraubbaren Knochenschrauben. Die Platte weist außerdem Schwächungen auf dergestalt, daß sie zur Anpassung verbogen werden kann.

Die Schwächung zur Herstellung der Verformbarkeit besteht erfindungsgemäß darin, daß eine Vielzahl von vorzugsweise runden Löchern vorgesehen ist, deren Durchmesser kleiner ist als der der Schraubenlöcher. Eine derartige Schwächung läßt sich relativ einfach herstellen und auch ggf. unterschiedlich vornehmen. Die Verteilung der Schraubenlöcher und der kleineren Löcher ist so gewählt, daß die Platte im Längsmittenbereich steifer als zum Längsrand hin ist.

Die Platte nach der Erfindung ist mithin so ausgebildet, daß sie entweder vor der Operation in Anpassung an die Kontur der Wirbelkörper vorgeformt oder vom Operateur unmittelbar während der Operation geformt werden kann. Anschließend wird sie mit Hilfe von Knochenschrauben am Wirbel bzw. Wirbelkörper fixiert, wobei Schraublöcher zum einen annähernd in der Mittenachse der Platte geformt sein können und auch zusätzlich zum Rand hin, der zur Anpassung an die Wirbelkörper entsprechend um die Längsachse abgebogen wird. Zwecks Anpassung an die Vertiefungen im Wirbelkörper wird die Platte außerdem um eine Querachse gebogen, so daß sie aufgrund der verschiedenen Biegungen eine erhebliche Stabilität erhält, die ausreicht, zum Beispiel für eine Primärversorgung frakturierter oder anderweitig defizienter Wirbel.

Die Anbringung der erfindungsgemäßen Platte erfolgt anterior, d.h. von vorn, so daß sie insbesondere für den Halswirbelbereich der Wirbelsäule geeignet ist. Die Anbringung ist relativ unproblematisch und äußerst platzsparend, so daß eine Beeinträchtigung des Patienten insoweit nicht gegeben ist. Die Knochenschrauben werden entweder nur im Wirbelkörper verankert oder auch in den Pedikeln des betreffenden Wirbels oder in diesen ausschließlich.

Die erfindungsgemäße Platte stellt einerseits eine ausreichende Verformbarkeit bereit und andererseits eine ausreichende Steifigkeit.

Damit die Platte durch Schraublöcher nicht so sehr geschwächt wird, ist die Anzahl naturgemäß zu begrenzen. Eine Ausgestaltung der Erfindung sieht daher vor, daß der Abstand oder das Vielfache des Abstands der Schraublöcher in Längsrichtung der Platte etwa dem Mittenabstand benachbarter Wirbelkörper entspricht, ggf. nach Verformung um eine Quer- bzw. Längsachse zur Anpassung an die Kontur der Wirbelkörper. Die Knochenschrauben lassen sich insbesondere im Halswirbelbereich leichter mittig in den Wirbelkörper einschrauben als im unteren oder oberen Bereich.

Die Länge der Platte hängt naturgemäß von der Größe des befallenen Bereiches ab. Unter Umständen reicht die Verwendung einer zwei Wirbelkörper abdeckenden Platte. Unter Umständen ist die Länge der Platte erheblich größer zu wählen. Damit nicht für jede Länge eine separate Platte gefertigt werden muß, sieht eine Ausgestaltung der Erfindung vor, daß die Platte von einem Endlosband abtrennbar ist. Der Chirurg kann daher während der Operation die Länge der Platte bestimmen, indem er sie in der gewünschten Menge von dem Vorrat abschneidet.

Eine andere Ausgestaltung der Erfindung sieht vor, daß die Schraublöcher in den Endbereichen der Platte länglich geformt sind und zum Ende hin eine kleiner werdende Breite aufweisen und die Lochränder eine Schrägung aufweisen, die mit der konischen Unterseite des Schraubenkopfes zusammenwirkt. Auf diese Weise kann durch das Zusammenwirken der konischen Kopfunterseiten der Schrauben mit den länglichen Löchern eine Kompressionskraft aufgebracht werden.

Schließlich sieht eine Ausgestaltung der Erfindung vor, daß an den Kanten der Platte kleine ohrartige Ansätze vorgesehen sind, die kleine Durchgangslöcher aufweisen. Mit den kleinen Löchern können zum Beispiel Drähte verbunden werden, um eine zusätzliche Fixierung zu erhalten.

Ausführungsbeispiele der Erfindung werden nachfolgend anhand von Zeichnungen näher erläutert.

Fig. 1 zeigt die Draufsicht auf eine erste Ausführungsform einer schematisch dargestellten Platte nach der Erfindung.

Fig. 2 zeigt die Draufsicht auf eine zweite Ausführungsform einer Platte nach der Erfindung.

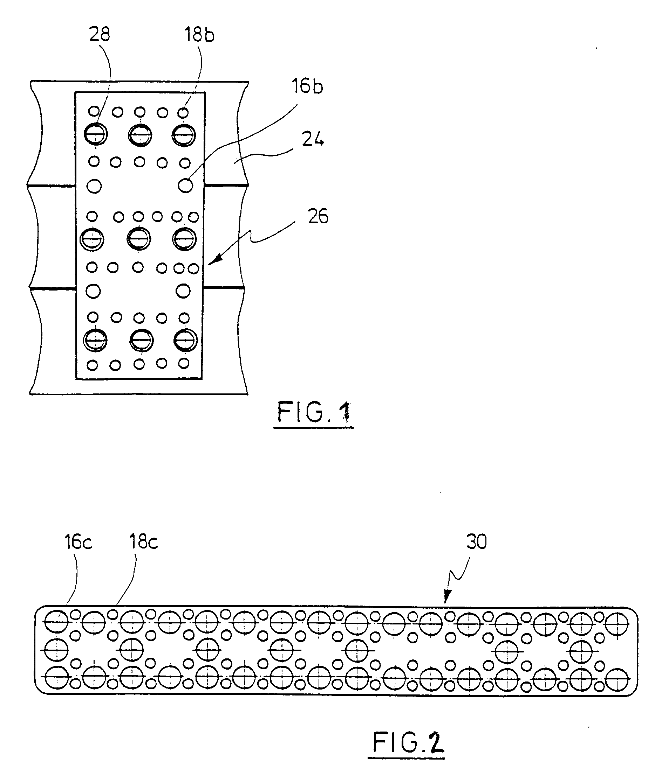

In Fig. 1 sind Wirbelkörper 24 von Halswirbeln angedeutet, die mit Hilfe einer Platte 26 miteinander verbunden sind. Der Aufbau der Platte 26 besitzt Querreihen von Schraubenlöchern 16b und Querreihen von Durchgangslöchern 18b, die einen kleineren Durchmesser aufweisen und lediglich Verformungszwecken dienen. Die Platte 26 ist, wie aus Fig. 1 nicht ohne weiteres ersichtlich, zum einen um die Längsachse gebogen, insbesondere im Randbereich und zusätzlich auch um Querachsen, um eine Anpassung an die Außenkontur der Wirbelkörper 24 zu erhalten. Lediglich die etwa in der Mitte der Wirbelkörper 24 liegenden Schraubenlöcher 16b sind von Knochenschrauben 28 durchsetzt.

Bei der Platte 30 nach Fig. 2 sind wiederum Querreihen von Schraublöchern 16c vorgesehen, wobei die Reihen mit drei Schraublöchern 16c mit Reihen von zwei Schraublöchern 16c abwechseln. Der Abstand zwischen den Reihen mit drei Schraublöchern 16c bzw. zwischen den Reihen mit zwei Schraublöchern 16c entspricht wiederum annähernd dem Mittenabstand benachbarter Wirbel der Halswirbelsäule. Zwischen den Schraublöchern sind wiederum Querreihen von Durchgangslöchern 18c mit kleinerem Durchmesser vorgesehen. Man erkennt, daß durch die Anordnung der Schraublöcher 16c die Platte 30 im Mittenbereich steifer ist als zu den Längsrändern hin. Dies wird noch verstärkt dadurch, daß der Abstand der mittleren beiden Löcher 18c größer ist als zwischen einem mittleren und einem außen liegenden Loch 18c einer Querreihe. Auf diese Weise wird eine relativ leichte Verformbarkeit im Randbereich erzielt, während der mittlere Bereich deutlich steifer ist.

Die Platte 30c kann endlos geformt werden, so daß der Chirurg die gewünschte Länge abschneidet.

## Patentansprüche

1. Stützplatte für die Korrektur und/oder die Stabilisierung traumatisierter oder defizienter Wirbel der menschlichen Wirbelsäule, insbesondere im Halswirbelbereich, die anterior an mindestens zwei benachbarten Wirbeln bzw. Wirbelkörpern (24) anbringbar ist und aus verformbarem, körperverträglichem Material besteht, wobei die Platte Schraubenlöcher (16b, 16c) aufweist zur Aufnahme von in die Wirbel bzw. Wirbelkörper (24) einschraubbaren Knochenschrauben (28) und die Schwächung (18b, 18c) so geformt ist, daß sie an die Kontur der Wirbelkörper (24) anpaßbar ist, dadurch gekennzeichnet, daß die Schwächung durch eine Vielzahl von vorzugsweise kreisrunden Löchern (18b, 18c) erfolgt, deren Durchmesser kleiner ist als der der Schraubenlöcher (16b, 16c) und die Verteilung der Schraubenlöcher (16b, 16c) so gewählt ist, daß die Platte (26, 30) im Längsmittenbereich steifer ist als zum Längsrand hin.

2. Stützmittel nach Anspruch 1, dadurch gekennzeichnet, daß der Abstand oder das Vielfache des Abstands der Schraublöcher (16b, 16c) in Längsrichtung der Platte (26, 30) etwa dem Mittenabstand benachbarter Wirbelkörper (24) entspricht, ggf. nach Verformung um eine Querachse bzw. Längsachse zwecks Anpassung an die Kontur der Wirbelkörper (24).

3. Stützmittel nach Anspruch 1 oder 2, dadurch gekennzeichnet, daß die Platte (30) von einem Endlosband abtrennbar ist.

4. Stützmittel nach einem der Ansprüche 1 bis 3, dadurch gekennzeichnet, daß die Schraublöcher (22) in den Endbereichen der Platte (20) länglich geformt sind mit zum Ende hin kleiner werdender Breite und die Lochränder eine Schrägung aufweisen, die mit der konischen Unterseite des Schraubenkopfes zusammenwirken.

5. Stützmittel nach einem der Ansprüche 1 bis 4, dadurch gekennzeichnet, daß an den Kanten der Platten ohrartige Ansätze vorgesehen sind, die kleine Durchgangslöcher aufweisen.

## Claims

1. A support plate for correcting and/or stabilizing injured or deficient vertebras of the human spine, in particular of the cervical vertebra region, which support plate is adapted to be attached anteriorly to at least a pair of adjacent vertebras (24) and is made of deformable, body-compatible material, the plate comprising screw holes (16b, 16c) for receiving bone screws (28) adapted to be screwed into the vertebras (24), and the weakening (18b, 18c) being formed such that it matingly conforms to the contour of the vertebras (24), characterized in that the weakening is obtained by a plurality of preferrably circular holes (18b, 18c) having a diameter which is smaller than that of the screw holes (16b, 16c), and that the arrangement of the screw holes (16b, 16c) is selected such that the plate is stiffer in the longitudinal center area than towards the longitudi-nal edge.

2. Support means according to claim 1, characterized in that the spacing or the multiple spacing of the screw holes (16b, 16c) in the longitudinal direction of the plate (26, 30) substantially corresponds to the spacing of the centers of adjacent vertebras (24), possibly after deformation about a transverse axis or, respectively, longitudinal axis to mate the contour of the vertebras (24).

3. Support means according to claim 1 or claim 2, characterized in that the plate (30) is cut from an endless band.

4. Support means according to any of claims 1 to 3, characterized in that the screw holes (22) in the end areas of the plate (20) are of elongated shape and of a width decreasing towards the end and that the edges of the holes are tapered so as to cooperate with the conical underside of the screw head.

5. Support means according to any of claims 1 to 4, charaterized in that ear-shaped projections are provided along the edges of the plate, the projections having small through-holes.

## Revendications

1. Plaque de soutien pour la correction et/ou la stabilisation de vertèbres traumatisées ou déficientes de la colonne vertébrale humaine, en particulier dans la zone des vertèbres cervicales, qui peut être appliquée antérieurement sur au moins deux vertèbres voisines ou corps de vertèbre (24) et qui est réalisée dans un matériau déformable, toléré par le corps, la plaque présentant des trous de vis (16b, 16c) destinés à loger des vis à os (28) à visser dans les vertèbres ou les corps de vertèbre (24) et l'affaiblissement (18b, 18c) étant formé de manière qu'elle puisse s'adapter au contour des corps de vertèbre (24), caractérisée en ce que l'affaiblissement s'effectue par un grand nombre de trous (18b, 18c) de préférence ronds, dont le diamètre est inférieur à celui des trous de vis (16b, 16c) et la répartition des trous de vis (16b, 16c) est choisie de manière que la plaque (26, 30) soit plus rigide dans la zone médiane longitudinale que vers le bord longitudinal.

2. Moyen de soutien selon la revendication 1, caractérisé en ce que l'écartement ou le multiple de l'écartement des trous de vis (16b, 16c) dans la direction longitudinale de la plaque (26, 30) correspond à peu près à l'écartement de centre à centre des corps de vertèbre (24) voisins, éventuellement après déformation autour d'un axe transversal ou axe longitudinal pour adaptation au contour des corps de vertèbre (24).

3. Moyen de soutien selon la revendication 1 ou 2, caractérisé en ce que la plaque (30) peut être découpée dans une bande sans fin.

4. Moyen de soutien selon l'une des revendications 1 à 3, caractérisé en ce que les trous de vis (22) sont formés longitudinalement dans les zones d'extrémité de la plaque (20) et présentent une largeur diminuant vers l'extrémité et les bords des trous présentent un chanfrein qui coopère avec la face inférieure conique de la tête de vis.

5. Moyen de soutien selon l'une des revendications 1 à 4, caractérisé en ce que sur les bords des plaques sont prévus des appendices en forme d'oreille qui présentent de petits trous de passage.
